# EUROPÄISCHE PATENTSCHRIFT

(11) **EP 3 606 513 B1**
(45) Veröffentlichungstag und Bekanntmachung des Hinweises auf die Patenterteilung: **23.08.2023**
(21) Anmeldenummer: 18716243.3
(22) Anmeldetag: 05.04.2018
(51) Int. Cl.: A61K 9/00, A61F 13/00, A61K 9/70

(54) **VERFAHREN ZUR HERSTELLUNG EINES, INSBESONDERE ORALEN, WIRKSTOFFLAMINATS UND WIRKSTOFFLAMINAT, INSBESONDERE ORALES WIRKSTOFFLAMINAT**
METHOD FOR PRODUCING AN, IN PARTICULAR ORAL, ACTIVE SUBSTANCE LAMINATE, AND ACTIVE SUBSTANCE LAMINATE, IN PARTICULAR ORAL ACTIVE SUBSTANCE LAMINATE
PROCÉDÉ POUR LA PRODUCTION D'UN STRATIFIÉ DE PRINCIPE ACTIF, EN PARTICULIER ORAL, ET STRATIFIÉ DE PRINCIPE ACTIF, EN PARTICULIER STRATIFIÉ DE PRINCIPE ACTIF ORAL

(30) Priorität: 06.04.2017 DE 102017107468
(43) Veröffentlichungstag der Anmeldung: 12.02.2020
(73) Patentinhaber: LTS Lohmann Therapie-Systeme AG, 56626 Andernach (DE)
(72) Erfinder: HOFFMANN, Michael, 56566 Neuwied (DE); KIRSTGEN, Elvira, 56564 Neuwied (DE); STEIN, Ralf-Ingo, 56269 Dierdorf (DE); WIEDERSBERG, Sandra, 06268 Steigra (DE); STÜMPER, Thomas, 53557 Bad Hönningen (DE)
(74) Vertreter: Meissner Bolte Partnerschaft mbB
(86) Internationale Anmeldenummer: PCT/EP2018/058761
(87) Internationale Veröffentlichungsnummer: WO 2018/185238

(56) Entgegenhaltungen:
- EP-A1- 2 564 938
- WO-A1-03/011248
- WO-A1-03/018071
- DE-A1- 19 826 592
- DE-U1-202008 017 304

## Beschreibung

Die Erfindung betrifft ein Verfahren zur Herstellung eines, insbesondere oralen, Wirkstofflaminats, das mindestens eine wirkstoffhaltige Schicht aufweist, das auf einem Substrat angeordnet ist, gemäß Patentanspruch 1. Außerdem betrifft die Erfindung ein Wirkstofflaminat, insbesondere ein orales Wirkstofflaminat, das insbesondere mit einem erfindungsgemäßen Verfahren hergestellt ist, wobei das orale Wirkstofflaminat mindestens eine wirkstoffhaltige Schicht aufweist, die auf einem Substrat angeordnet ist, gemäß Patentanspruch 9.

Orale Wirkstofflaminate bzw. orale Wirkstofffilme (Oral Thin Films) sind dünne, wirkstoffhaltige Filme, die direkt in den Mundraum gelegt oder an die Mundschleimhaut angelegt werden und sich dort in kurzer Zeit auflösen. Beispielhafte Auflösezeiten betragen zwischen einer und dreißig Minute(n). Bei transmukosalen Filmen gelangt der Wirkstoff über die Mundschleimhaut in den Blutkreislauf, ohne den Magen-Darm-Trakt passieren zu müssen.

Orale Wirkstofflaminate bzw. Wirkstoff-Filme sind dünne, flexible Arzneimittelträger. Man legt sie z. B. auf oder unter die Zunge und lässt diese zergehen. Außerdem ist es möglich, die Oral Thin Films an der Mundschleimhaut, insbesondere an der inneren Wangentasche, anzuhaften. Die sehr gute Durchblutung der Mundschleimhaut sorgt für einen unmittelbaren Übergang des Wirkstoffs in den Blutkreislauf.

Des Weiteren ist es bekannt, zahnmedizinische Prophylaxe und/oder Munderfrischungsprodukte als orale Wirkstofflaminate auszubilden.

DE 20 2008 017304 U1 beschreibt einen oral zerfallenden Film und ein Verfahren zu dessen Herstellung.

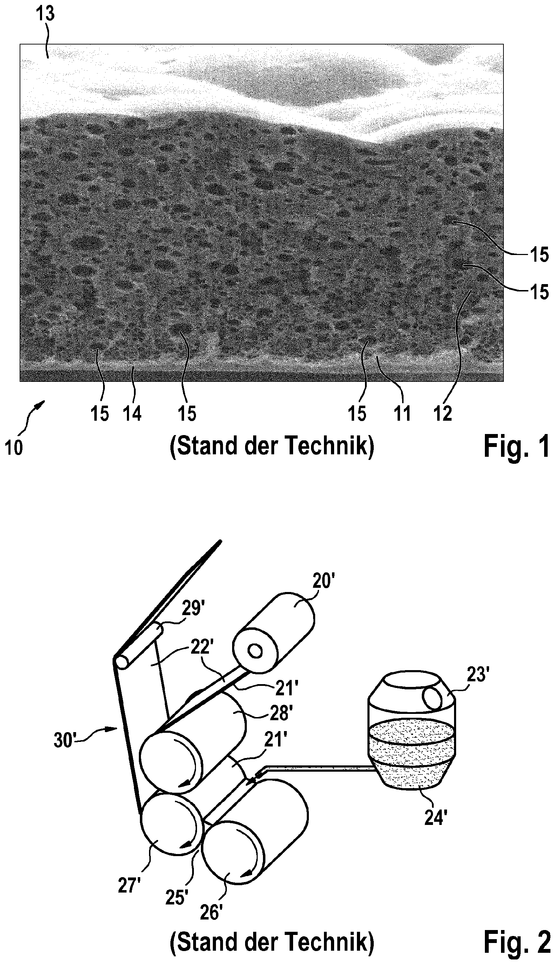

In Fig. 1 wird beispielhaft ein derartiges Prophylaxe-Laminat dargestellt. Es handelt sich hierbei um ein etwa 70 µm dickes Produkt 10, wobei Menthol in Form öliger Tröpfchen 15 in einer Matrix 12 eingelagert ist. Die Oberfläche 13 des Produktes 10 ist, wie deutlich zu erkennen ist, äußerst uneben. Dies ist eine Folge der bislang bekannten Herstellungsverfahren bzgl. oraler Wirkstofflaminate. Die Unterseite 14 ist hingegen sehr glatt bzw. eben ausgebildet, wobei diese glatte bzw. ebene Ausbildung der Unterseite 14 auf die verwendete Trägerfolie 11 zurückzuführen ist. Auch wenn pro Flächeneinheit die gleiche Menge an Wirkstoff enthalten ist, so ist die Matrix 12 trotzdem im Querschnitt relativ unhomogen ausgebildet. Zu erkennen ist, dass in Richtung der Trägerfolie 11 mehr Tröpfchen 15 ausgebildet sind, wobei diese Tröpfchen einen kleineren Durchmesser aufweisen als die Tröpfchen 15, die im Bereich der Oberfläche 13 ausgebildet sind.

Die beiden wichtigsten Techniken, die verwendet werden, um die orale Filme herzustellen, sind Lösungsmittelgießen und Schmelzextrusion.

Bisherige Verfahren zum Herstellen oraler Wirkstofflaminate rufen des Weiteren das Problem hervor, wonach das Flächengewicht von wirkstoffhaltigen Schichten, insbesondere bei mehrschichtigen Systemen, nicht ausreichend konstant ist. Ein beispielhaftes bekanntes Verfahren ist das "Standard Roll Coating". Das Flächengewicht einer wirkstoffhaltigen Schicht kann meist nicht separat, sondern nur im Verbund, d. h. als Summe, mit den wirkstofffreien Schichten bestimmt werden. Das Gesamtflächengewicht eines oralen Wirkstofflaminats beinhaltet sowohl Schwankungen im Flächengewicht der wirkstoffhaltigen Schicht als auch der wirkstofffreien Schicht(en).

Beim Auftragen einer wirkstoffhaltigen Schicht mittels "Standard Roll Coating" wird die zu beschichtende wirkstoffhaltige Masse auf eine wirkstofffreie oder ebenfalls wirkstoffhaltige Schicht bzw. auf ein wirkstofffreies oder wirkstoffhaltiges Substrat direkt aufgetragen. Schwankungen hinsichtlich der Dicke des Substrates bzw. hinsichtlich des Flächengewichts des Substrates bzw. der wirkstofffreien oder wirkstoffhaltigen Schicht können sich somit direkt auf die Schwankungen im vollständigen oralen Wirkstofflaminat auswirken.

In EP 1 087 759 B1 wird eine sog. "flüssig auf flüssig"-Beschichtung beschrieben. Hierbei können mehrschichtige Laminate in einer geschlossenen Folge äußerst wirtschaftlicher Herstellungsprozesse erarbeitet werden. Zunächst wird eine unter Verarbeitungsbedingungen fließfähige erste Masse als erste Schicht aufgetragen und anschließend eine weitere, unter Verarbeitungsbedingungen fließfähige Masse als weitere Schicht aufgetragen.

In Fig. 2 ist ein beispielhaftes Standard-Beschichtungsverfahren dargestellt. Ein aufgewickeltes Substratmaterial 20' wird mit Hilfe einer optional gummierten Transportrolle 28' abgerollt und in einen von einer ersten Rolle 26' und einer zweiten Rolle 27' gebildeten Spalt 25' geführt. Auf die Oberseite 21' des Substrats 20' wird im Bereich des Spalts 25' die wirkstoffhaltige Masse 24' aufgetragen. Der Auftrag der wirkstoffhaltigen Masse 24' auf die Oberseite 21' erfolgt direkt. Der Wirkstoff 24' kommt demnach mit der Oberfläche der zweiten Rolle 27' nicht in Kontakt. Mit Hilfe der zweiten Rolle 27' wird das beschichtete Substrat 20' zusammen mit der aufgetragenen wirkstoffhaltigen Masse 24' als Zwischenlaminat 30' beispielsweise zu einer weiteren Transportrolle 29' geführt. Schwankungen hinsichtlich des Flächengewichts des Substrates 20' wirken sich somit direkt als Schwankungen im Zwischenlaminat 30' bzw. im vollständigen oralen Wirkstofflaminat aus.

Es ist somit Aufgabe der vorliegenden Erfindung ein Verfahren zur Herstellung eines, insbesondere oralen, Wirkstofflaminates anzugeben, wonach eine wirkstoffhaltige Schicht unabhängig vom Flächengewicht bzw. den Schichtdicken bzw. Schichtdickenschwankungen des Substrates hergestellt bzw. aufgetragen werden kann.

Erfindungsgemäß wird die erläuterte Aufgabe im Hinblick auf das Verfahren zur Herstellung eines, insbesondere oralen, Wirkstofflaminats durch den Gegenstand des Patentanspruchs 1 und im Hinblick auf das Wirkstofflaminat, insbesondere das orale Wirkstofflaminat, durch den Gegenstand des Patentanspruchs 9 gelöst.

Die Erfindung beruht auf dem Gedanken, ein Verfahren zur Herstellung eines, insbesondere oralen, Wirkstofflaminats, das mindestens eine wirkstoffhaltige Schicht aufweist, die auf einem Substrat angeordnet ist, anzugeben. Das erfindungsgemäße Verfahren umfasst folgende Schritte:
a) Bereitstellen eines Substrats mit einer Oberseite und einer Unterseite;
b) Applizieren einer wirkstoffhaltigen Masse in einen Spalt, der von einer ersten drehenden Rolle und einer zweiten drehenden Rolle gebildet wird;
c) Transportieren des Substrats mittels einer dritten drehenden Rolle zur zweiten Rolle derart, dass die wirkstoffhaltige Masse von der zweiten Rolle auf die Oberseite des Substrats im Form einer wirkstoffhaltigen Schicht aufgetragen wird;
d) Transportieren eines Zwischenlaminats, das aus dem Substrat und der wirkstoffhaltigen Schicht gebildet wird, zu einer Trocknungsvorrichtung;
e) Trocknen des Zwischenlaminats, insbesondere der wirkstoffhaltigen Schicht, wobei die Rotationsgeschwindigkeit der ersten drehenden Rolle 0,0 m/min - 0,5 m/min beträgt und der zweiten drehenden Rolle 0,8 m/min - 2,3 m/ min beträgt und der dritten drehenden Rolle 0,5 m/min - 1,5 m/min beträgt, und der Spalt zwischen der ersten drehenden Rolle und der zweiten drehenden Rolle eine Breite von 0,2 mm - 0,9 mm aufweist und das Zwischenlaminat in der Trocknungsvorrichtung mit einer Temperatur von 30°C - 120°C beaufschlagt wird.

Die wirkstoffhaltige Masse wird erfindungsgemäß mit einer konstanten Dicke bzw. einem konstanten Flächengewicht und vom Substrat unabhängig, von der zweiten Rolle auf die Oberseite des Substrats unter Bildung einer wirkstoffhaltigen Schicht aufgetragen.

Die erste und die zweite Rolle sind vorzugsweise nebeneinander angeordnet, sodass in vertikaler Richtung zwischen den beiden Rollen ein Spalt gebildet werden kann, in den eine wirkstoffhaltige Masse appliziert werden kann. Mit anderen Worten wird beispielsweise ausgehend von einem Container, der mit einer wirkstoffhaltigen Masse befüllt ist, in den Spalt die wirkstoffhaltige Masse eingefüllt. Die wirkstoffhaltige Masse wird entlang der zweiten Rolle in vertikaler Richtung nach unten transportiert. Um einen derartigen Transport zu gewährleisten ist es beispielsweise möglich, an der ersten Rolle einen Rakel bzw. ein Streichmesser vorzusehen, sodass die wirkstoffhaltige Masse lediglich entlang der zweiten Rolle nach unten transportiert wird.

Oberhalb oder unterhalb der zweiten Rolle ist eine dritte Rolle angeordnet. Mittels dieser dritten drehenden Rolle wird ein Substrat zur zweiten Rolle transportiert. Das Substrat kann bahnförmig vorliegen. Des Weiteren ist es denkbar, dass das Substrat im aufgerollten Zustand vorliegt und mit Hilfe der dritten drehenden Rolle abgerollt und zur zweiten Rolle transportiert wird. Da die dritte drehende Rolle über- oder unterhalb der zweiten drehenden Rolle angeordnet ist, kann der Wirkstoff bzw. die wirkstoffhaltige Masse von der zweiten Rolle auf die Oberseite des Substrats aufgetragen werden. Der Wirkstoff bzw. die wirkstoffhaltige Masse wird zunächst mit konstanter Dicke auf die Oberfläche der zweiten drehenden Rolle aufgetragen und mit Hilfe der Rollenoberfläche transportiert. Der Auftrag des Wirkstoffs bzw. der wirkstoffhaltigen Masse auf das Substrat erfolgt somit nicht direkt von einem Behälter aus, sondern indirekt durch ein Abdrucken des auf der Rollenoberfläche befindlichen Wirkstoffs auf die Oberseite des Substrats.

Der Wirkstoff liegt in der wirkstoffhaltigen Masse vorzugsweise in gelöster Form vor. Die wirkstoffhaltige Masse kann als Emulsion vorliegen. Auch das Vorliegen des Wirkstoffs in dispergierter Form in der wirkstoffhaltigen Masse ist möglich. Die wirkstoffhaltige Masse umfasst vorzugsweise ein Lösungsmittel. Das Lösungsmittel sorgt für die Fließfähigkeit der wirkstoffhaltigen Masse. Das Lösungsmittel wird vorzugsweise im Schritt e), nämlich im Trocknungsschritt, entfernt. Mit anderen Worten wird das Zwischenlaminat, insbesondere die wirkstoffhaltige Schicht, durch das Entfernen des Lösungsmittels getrocknet. Neben Wasser können auch andere Lösungsmittel verwendet werden, z.B. Alkohole wie Methanol, Ethanol, Propanol, oder Lösungsmittelgemische, z. B. Wasser-AlkoholGemische.

Als Substrat ist ein derartiges Substrat zu verstehen, das erst aufgrund des erfindungsgemäßen Verfahrens mit ausreichendem medikamentösem Wirkstoff beschichtet wird. Das Substrat ist wirkstofffrei ausgebildet sein. Das Substrat kann im einfachsten Fall aus Cellulose ohne Wirkstoff bestehen.

In einer bevorzugten Ausführungsform der Erfindung weist das Substrat die in Tabelle 1 angegebene Zusammensetzung auf. Die angegebenen Mengen beziehen sich auf die Herstellung von 150 kg Flüssigmasse des zu produzierenden Substrats.

**Tabelle 1**

| **Bestandteil** | **Gewichts-Prozent (w/w)** | **Menge (kg)** |
|---|---|---|
| Aufbereitetes Wasser | 77.50 | 116.249 |
| Konservierungsstoff(e) | 0.24 | 0.356 |
| Hilfsstoff(e) | 0.11 | 0.169 |
| Antioxidationsmittel | 0.01 | 0.018 |
| Matrixmaterial(ien) | 21.30 | 31.960 |
| Füllstoff(e) | 0.55 | 0.827 |
| Süßungs- und Geschmacksstoff(e) | 0.28 | 0.422 |
| Release Liner *^{a}* | | |
| Gesamt | 100.0 | 150.00 |
| *a* = *Bestandteil, der während des Herstellungsprozesses entfernt wird.* | | |

Das Substrat bildet zusammen mit dem aufgetragenen Wirkstoff, also der gebildeten wirkstoffhaltigen Schicht, ein Zwischenlaminat.

Das Substrat kann auf der Unterseite mit einer Trägerfolie, insbesondere lösbar, verbunden sein. Die Trägerfolie dient beispielsweise als Prozessfolie mit deren Hilfe das erfindungsgemäße Verfahren vereinfacht durchgeführt werden kann. Insbesondere der Transport des beschichteten Substrates bzw. des Zwischenlaminats wird mit Hilfe einer Trägerfolie erleichtert.

Das wirkstofffreie Substrat kann aus zwei wirkstofffreien Schichten gebildet sein. In diesem Fall bildet beispielsweise eine erste wirkstofffreie Schicht die Oberseite des Substrats und eine zweite Schicht die Unterseite des Substrats. Es ist möglich, dass zwischen der ersten Schicht und der zweiten Schicht mindestens eine weitere wirkstofffreie Schicht ausgebildet ist.

Beispielsweise kann es sich bei dem Wirkstoff der wirkstoffhaltigen Schicht um Naloxan und/oder Fentanyl handeln.

Die wirkstoffhaltige Schicht kann eine mucoadhäsive Schicht sein. In einer bevorzugten Ausführungsform der Erfindung weist die wirkstoffhaltige bzw. mucoadhäsive Schicht die in Tabelle 2 angegebene Zusammensetzung auf. Die angegebenen Mengen beziehen sich auf die Herstellung von 65 kg Flüssigmasse der zu produzierenden wirkstoffhaltigen bzw. mucoadhäsiven Schicht.

**Tabelle 2**

| **Bestandteil** | **Gewichts-Prozent (w/w)** | **Menge (kg)** |
|---|---|---|
| Fentanyl citrat | 0.86 | 0.561 |
| Aufbereitetes Wasser | 88.82 | 57.733 |
| Weichmacher | 0.48 | 0.311 |
| Konservierungsstoff(e) | 0.18 | 0.117 |
| Farbstoff(e) | 0.01 | 0.008 |
| pH- Wert Modulator(en) | 1.31 | 0.855 |
| Antioxidationsmittel | 0.01 | 0.004 |
| Matrixmaterial(ien) | 8.32 | 5.411 |
| **Gesamt** | **100.0** | **65.00** |

Die aufgeführten Matrixmaterialien können wasserlösliche oder wasserquellbare Polymere sein, z.B. Cellulose und/oder deren Derivate.

Die erste Rolle, die zweite Rolle und die dritte Rolle drehen in der gleichen Rotationsrichtung. Mit anderen Worten drehen die erste, die zweite und die dritte Rolle gleichzeitig im Uhrzeigersinn oder gleichzeitig entgegen des Uhrzeigersinns.

Die Rotationsgeschwindigkeit der ersten drehenden Rolle beträgt erfindungsgemäß 0,0 m/min - 0,5 m/min.

Vorzugsweise kann die Rotationsgeschwindigkeit der ersten drehenden Rolle 0,1 m/min - 0,3 m/min betragen. Vorzugsweise weist die Rotationsgeschwindigkeit der ersten drehenden Rolle einen Wert der genannten Wertebereiche auf, wobei die Rotationsgeschwindigkeit während des erfindungsgemäßen Verfahrens konstant ist.

Die Rotationsgeschwindigkeit der zweiten drehenden Rolle beträgt erfindungsgemäß 0,8 m/min - 2,3 m/min.

Vorzugsweise kann die Rotationsgeschwindigkeit der zweiten drehenden Rolle 1,0 m/min - 1,5 m/min betragen. Vorzugsweise weist die Rotationsgeschwindigkeit der zweiten drehenden Rolle einen Wert der genannten Wertebereiche auf, wobei die Rotationsgeschwindigkeit während des erfindungsgemäßen Verfahrens konstant ist.

Die Rotationsgeschwindigkeit der dritten drehenden Rolle beträgt erfindungsgemäß 0,5 m/min - 1,5 m/min.

Vorzugsweise kann die Rotationsgeschwindigkeit der dritten drehenden Rolle 0,7 m/min - 1,0 m/min betragen. Vorzugsweise weist die Rotationsgeschwindigkeit der dritten drehenden Rolle einen Wert der genannten Wertebereiche auf, wobei die Rotationsgeschwindigkeit während des erfindungsgemäßen Verfahrens konstant ist.

Es hat sich gezeigt, dass bei den genannten Rotationsgeschwindigkeiten ein besonders gleichmäßiges Flächengewicht hinsichtlich der wirkstoffhaltigen Schicht auf das Substrat, insbesondere auf die erste wirkstofffreie Schicht, aufgetragen werden kann.

Der Spalt zwischen der ersten drehenden Rolle und der zweiten drehenden Rolle weist erfindungsgemäß eine Breite von 0,2 mm - 0,9 mm auf.

Vorzugsweise kann der Spalt zwischen der ersten drehenden Rolle und der zweiten drehenden Rolle eine Breite von 0,6 mm aufweisen. Die Spaltbreite ist vorzugsweise während des erfindungsgemäßen Verfahren konstant und weist einen Wert des genannten Wertebereiches auf.

Es hat sich gezeigt, dass mit Hilfe eines derartigen Spaltmaßes eine besonders gleichmäßige Beschichtung des Substrats mit einer wirkstoffhaltigen Schicht möglich ist.

Das Zwischenlaminat wird zu einer Trocknungsvorrichtung transportiert, wobei das Zwischenlaminat in der Trocknungsvorrichtung mit einer Temperatur von 30 °C - 120 °C beaufschlagt wird.

Vorzugsweise wird das Zwischenlaminat in der Trocknungsvorrichtung mit einer Temperatur von 60 °C - 90 °C, insbesondere von 75 °C, beaufschlagt.

Vorzugsweise wird das Zwischenlaminat mit einer Geschwindigkeit, dem sogenannten "web speed", von 0,7 m/min - 0,9 m/min, insbesondere von 0,8 m/min, transportiert. Der sog. "web speed" ist die Prozessgeschwindigkeit. Der sog. "web speed" kann auch als Transportgeschwindigkeit des herzustellenden Wirkstofflaminats bezeichnet werden. Diese Geschwindigkeit ist während des gesamten Verfahrens gleich und weist einen Wert des genannten Wertebereiches auf.

Sofern das Zwischenlaminat mit einer derartigen Temperatur beaufschlagt wird, kann ein Zwischenlaminat erzeugt werden, das hinsichtlich der auf der wirkstoffhaltigen Schicht gebildeten Oberfläche ein besonders ebenes bzw. glattes Ergebnis erzeugt.

Bei der Trocknungsvorrichtung kann es sich beispielsweise um einen Trocknungskanal handeln. Der Trocknungskanal kann mehrere Zonen, insbesondere mehrere Temperaturzonen aufweisen. Vorzugsweise weist der Trocknungskanal mindestens zwei, insbesondere mindestens drei, insbesondere mindestens vier, insbesondere mindestens fünf, insbesondere mindestens zehn, insbesondere mindestens dreizehn, Trocknungszonen auf. Mit einer steigenden Anzahl von Trocknungszonen kann das Zwischenlaminat schneller getrocknet werden. Das Verfahren kann somit mit steigender Anzahl von Trocknungszonen schneller durchgeführt werden.

Die Trocknungsvorrichtung kann mindestens eine Luftdüse aufweisen, sodass das Zwischenlaminat mit erwärmter Luft mit einer Temperatur von 30 °C - 120 °C, insbesondere von 60 °C - 90 °C, insbesondere von 75 °C, beaufschlagt wird.

Sofern es sich bei der Trocknungsvorrichtung um einen Trocknungskanal mit mehreren Trocknungszonen handelt, kann die Trocknungsvorrichtung mehrere Luftdüsen aufweisen, wobei die erwärmte Luft der Luftdüsen unterschiedliche Temperaturen erzeugt. Vorzugsweise kann vorgesehen sein, dass innerhalb des Trocknungskanals zu Beginn in den Trocknungszonen mit geringen Temperaturen und im weiteren Verlauf des Trocknungskanals in den Trocknungszonen mit höheren Temperaturen das Zwischenlaminat getrocknet wird.

In einer weiteren bevorzugten Ausführungsform des erfindungsgemäßen Verfahrens wird das Zwischenlaminat derart in den Trocknungskanal eingeführt, dass die wirkstoffhaltige Schicht in Richtung der mindestens einen Luftdüse weist.

Beispielweise ist die mindestens eine Luftdüse oberhalb der wirkstoffhaltigen Schicht angeordnet. Die wirkstoffhaltige Schicht wird somit von oben mit warmer Luft beaufschlagt. Somit wird die wirkstoffhaltige Schicht direkt mit höherer Temperatur als die Unterseite des Substrats beaufschlagt.

In einer alternativen Ausführungsform ist es möglich, dass Trocknungseinrichtungen, insbesondere Luftdüsen, unterhalb des Substrats angeordnet sind.

In einer weiteren Ausführungsform der Erfindung ist das Verfahren des Weiteren gekennzeichnet durch den Schritt
f) Führen des getrockneten Zwischenlaminats durch Laminierwalzen und Bilden des Wirkstofflaminats.

Zwei Laminierwalzen rotieren vorzugsweise gegenläufig bzw. in zwei verschiedenen Rotationsrichtungen. Aufgrund dessen wird das Zwischenlaminat verfestigt. Die beiden Laminierrollen können des Weiteren beheizt sein.

Der weitere Schritt
g) betrifft das Aufrollen des getrockneten Zwischenlaminats, also das Aufrollen des Wirkstofflaminats.

In einer aufgerollten Form kann das Wirkstofflaminat zu einer weiteren Vorrichtung transportiert werden. Des Weiteren ist es möglich, dass das aufgerollte getrocknete Wirkstofflaminat in einer anderen Betriebsstätte verwendet wird.

In einer Ausführungsform der Erfindung können weitere Verfahrensschritte, nämlich
h) Abrollen des Wirkstofflaminats;
i) optionales Entfernen der Trägerfolie; und
j) Zerteilen des Wirkstofflaminats in eine Vielzahl von Wirkstofflaminatabschnitte,
durchgeführt werden.

Alternativ ist es möglich, dass die Trägerfolie bereits vor dem Schritt g), also vor dem Aufrollen des Wirkstofflaminats, von dem Wirkstofflaminat entfernt wird. Das Zerteilen des Wirkstofflaminats in eine Vielzahl von Wirkstofflaminatabschnitte kann beispielsweise durch Schneiden und/oder Stanzen und/oder Perforieren und anschließendes Reißen erfolgen. Die einzelnen Wirkstofflaminatabschnitte bilden gebrauchsfertige orale Wirkstofflaminate bzw. orale Wirkstofffilme, die verpackt werden können.

Mit Hilfe des erfindungsgemäßen Verfahrens kann ein konstanter und präziser Wirkstoffgehalt in einem oralen Wirkstofflaminat hergestellt werden. Insbesondere ist es möglich, eine wirkstoffhaltige Schicht auf ein wirkstofffreies oder wirkstoffhaltiges Substrat mit konstanter und präziser Dicke bzw. mit konstantem und präzisem Flächengewicht aufzutragen.

Prinzipiell wäre es möglich, die wirkstoffhaltige Schicht direkt auf einen Träger zu beschichten und anschließend die wirkstofffreie Schicht oder Schichten auf der wirkstoffhaltigen Schicht zu beschichten. Eine Änderung der Beschichtungsreihenfolge ruft allerdings hervor, dass die wirkstoffhaltige Schicht mehrmals durch den Trockenkanal geführt werden müsste. Der Wirkstoff kann unter Umständen den hohen Temperaturen nicht standhalten, sodass die vorher beschriebene Reihenfolge der Verfahrensschritte als optimaler zu beurteilen ist.

Ein nebengeordneter Aspekt der Erfindung betrifft ein Wirkstofflaminat, insbesondere ein orales Wirkstofflaminat, das insbesondere mit dem erläuterten erfindungsgemäßen Verfahren hergestellt ist, wobei das Wirkstofflaminat mindestens eine wirkstoffhaltige Schicht aufweist, die auf einem Substrat angeordnet ist.

Bei einer besonders bevorzugten Ausführungsform der Erfindung handelt es sich bei dem Wirkstofflaminat um ein orales Wirkstofflaminat.

Synonyme oder weitere Begrifflichkeiten, die ein orales Wirkstofflaminat beschreiben sind beispielsweise thin-film (Dünnfilm), oral film (oraler Film), wafer (Wafer), oral strip (oraler Streifen), orodispersible film (Schmelzfilm), oral thin film (oraler Wirkstofffilm), oral soluble film (oral löslicher Film), dissofilms, buccal soluble film (wangenlöslicher Film), mucoadhesive film (mucoadhäsiver Film - an Schleimhaut haftender Film), buccal film (an der Wange befindlicher Film) und transmucosal film (transmukosaler Film). Das erfindungsgemäße orale Wirkstofflaminat kann als einer der beschriebenen Filme/Laminate ausgebildet sein.

Die wirkstoffhaltige Schicht bildet mindestens eine der beiden äußeren Oberflächen des oralen Wirkstofflaminats. Das Substrat ist wirkstofffrei ausgebildet.

Als wirkstofffreies Substrat ist ein derartiges Substrat zu verstehen, das erst aufgrund des erfindungsgemäßen Verfahrens mit ausreichendem medikamentösem Wirkstoff beschichtet wird.

Erfindungsgemäß weist die wirkstoffhaltige Schicht ein von der Dicke und/oder dem Flächengewicht des Substrats unabhängig konstantes Flächengewicht auf.

Der Wirkstoff ist ein pharmazeutischer Wirkstoff, nämlich ein Analgetikum, insbesondere Fentanyl und/oder Buprenorphin und/oder Opioidantagonist, sein. Bei einem Opioidantagonist kann es sich vorzugsweise um Naloxon handeln. Buprenorphin liegt vorzugsweise als Salz oder Base vor.

Die wirkstoffhaltige Schicht kann ein Flächengewicht von 40 g/m² - 100 g/m², insbesondere von 50 g/m² - 70 g/m², insbesondere von 55 g/m² - 65 g/m², insbesondere von 60 g/m², aufweisen.

Das Substrat ist beispielweise aus einer Schicht gebildet. Vorzugsweise ist das Substrat aus mindestens zwei Schichten gebildet. Bei den mindestens zwei Schichten handelt es sich um wirkstofffreie Schichten. Das Substrat kann aus einem Polymer und/oder einem wasserquellbaren Material und/oder einem wasserlöslichen Material bestehen. Bzgl. einer bevorzugten Zusammensetzung des Substrats, wird auf die Tabelle 1 verwiesen.

Das wirkstofffreie Substrat kann ein Flächengewicht von 100 g/m² - 300 g/m², insbesondere von 240 g/m² - 280 g/m², insbesondere von 250 g/m² - 270 g/m², insbesondere von 262 g/m², aufweisen.

Die wirkstofffreien Schichten können jeweils ein Flächengewicht von 80 g/m² - 180 g/m², insbesondere von 100 g/m² - 160 g/m², insbesondere von 120 g/m² - 140 g/m², insbesondere von 131 g/m², aufweisen.

Auf der zur wirkstoffhaltigen Schicht gegenüberliegenden Seite des wirkstofffreien Substrats kann eine Trägerfolie ausgebildet sein.

Die wirkstoffhaltige Schicht des Wirkstofflaminat bzw. des gebildeten Oral Thin Films haftet bei der Benutzung an der Mundschleimhaut des Verwenders. Die Haftung des Oral Thin Films erfolgt mit anderen Worten aufgrund von Mucoadhäsion. Durch die Mundschleimhaut wird der Wirkstoff der wirkstoffhaltigen Schicht vom Verwender aufgenommen. Bzgl. einer bevorzugten Zusammensetzung der wirkstoffhaltigen Schicht bzw. einer mucoadhäsiven Schicht, wird auf die Tabelle 2 verwiesen.

Die wirkstoffhaltige Masse kann eine Viskosität von 30 dPas - 60 dPas, insbesondere von 40 dPas - 50 dPas, insbesondere von 45 dPas, ausweisen.

Eine wirkstofffreie Masse kann eine Viskosität von 150 dPas - 250 dPas, insbesondere von 180 dPas - 230 dPas, insbesondere von 200 dPas, aufweisen. Die wirkstofffreie Masse kann zur Herstellung einer wirkstofffreien Schicht des Substrats dienen.

Die Erfindung wird im Folgenden anhand von Ausführungsbeispielen unter Bezugnahme auf die beigefügten schematischen Zeichnungen näher erläutert.

Darin zeigen:
- Fig. 1: einen Querschnitt durch einen Oral Thin Film gemäß Stand der Technik;
- Fig. 2: eine schematische Darstellung hinsichtlich eines aus dem Stand der Technik bekannten Beschichtungsverfahrens;
- Fig. 3: eine schematische Darstellung hinsichtlich des erfindungsgemäßen Verfahrens; und
- Fig. 4: einen Querschnitt durch ein erfindungsgemäßes orales Wirkstofflaminat.

In der nachfolgenden Beschreibung werden für gleiche und gleichwirkende Teile dieselben Bezugsziffern verwendet.

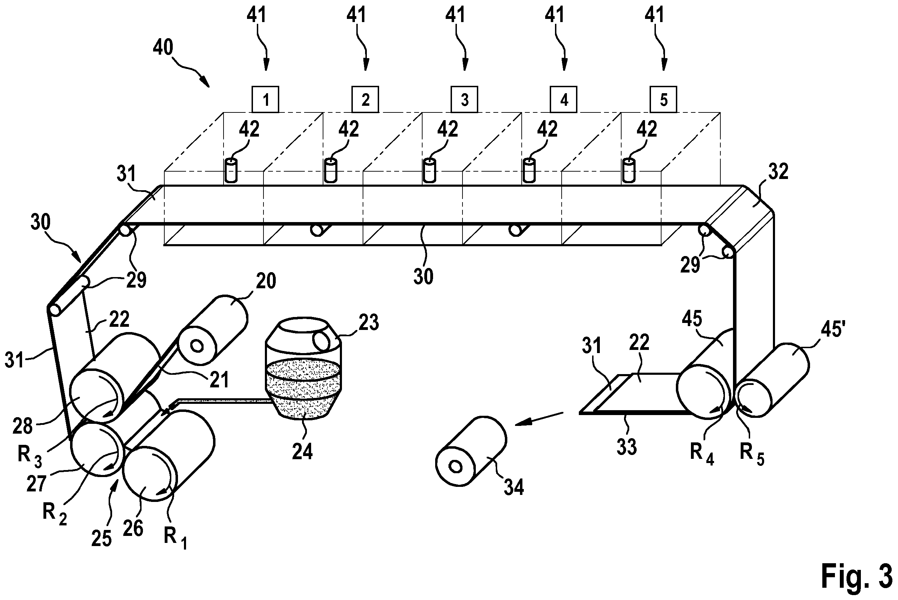

Fig. 3 zeigt schematisch den Verfahrensablauf hinsichtlich des erfindungsgemäßen Verfahrens zur Herstellung eines oralen Wirkstofflaminats. Insbesondere werden die einzelnen Stationen des erfindungsgemäßen Verfahrens dargestellt.

Ein, insbesondere wirkstofffreies, Substrat 20 mit einer Oberseite 21 und einer Unterseite 22 wird in aufgerollter Form bereitgestellt. Des Weiteren ist ein Behälter 23 zu erkennen, indem sich eine wirkstoffhaltige Masse 24 befindet. Die wirkstoffhaltige Masse 24 wird zu einem Spalt 25 transportiert. Der Spalt 25 wird von einer ersten drehenden Rolle 26 und einer zweiten drehenden Rolle 27 gebildet. Der Spalt 25 zwischen der ersten drehenden Rolle 26 und der zweiten drehenden Rolle 27 weist beispielsweise eine Breite von 0,6 mm auf.

Die erste drehende Rolle 26 und die zweite drehende Rolle 27 sind in horizontaler Richtung in etwa nebeneinander, insbesondere parallel, angeordnet, sodass ein im Wesentlichen vertikal verlaufender Spalt 25 gebildet werden kann. Eine dritte drehende Rolle 28 ist vertikal oberhalb der zweiten drehenden Rolle 27 angeordnet. In einer alternativen Ausführungsform des Verfahrens kann sich die dritte drehende Rolle 28 auch unterhalb der zweiten drehenden Rolle 27 befinden.

Um zu gewährleisten, dass die wirkstoffhaltige Masse 24 entlang der zweiten Rolle 27 in Richtung der dritten Rolle 28 transportiert wird, kann an der ersten drehenden Rolle 26 ein Streichmesser bzw. Rakel (nicht dargestellt) ausgebildet sein. Das Substrat 20 wird mit Hilfe der dritten drehenden Rolle 28 zur zweiten drehenden Rolle 27 derart transportiert, dass der Wirkstoff von der Oberfläche der zweiten Rolle 27 auf die Oberseite 21 des Substrats 20 aufgetragen wird.

Die Rotationsrichtung R₁ der ersten Rolle 26 und die Rotationsrichtung R₂ der zweiten Rolle 27 sowie die Rotationsrichtung R₃ der dritten Rolle 28 stimmen überein. Die Rotationsrichtungen R₁, R₂ und R₃ sind somit gleich. Die Rotationsgeschwindigkeiten betragen im dargestellten Beispiel für die erste Rolle (26) 0,0 m/min - 0,5 m/min, für die zweite Rolle (27) 0,8 m/min - 2,3 m/min und für die dritte Rolle (28) 0,5 m/min - 1,5 m/min. Die Rotationsgeschwindigkeiten der ersten Rolle 26, der zweiten Rolle 27 und der dritten Rolle 28 weisen einen Wert der genannten Wertebereiche auf, wobei alle Rotationsgeschwindigkeiten während des Durchführens des erfindungsgemäßen Verfahrens konstant sind.

Das Zwischenlaminat 30, das aus dem Substrat 20 sowie der wirkstoffhaltigen Schicht 31 gebildet ist, wird mit Hilfe von Transportrollen 29 zu einer Trocknungsvorrichtung 40 transportiert. Bei der Trocknungsvorrichtung 40 handelt es sich um einen Trocknungskanal mit beispielhaft fünf Trocknungszonen 41. Jede Trocknungszone weist eine Luftdüse 42 auf. Mit Hilfe der Luftdüsen 42 werden in den fünf Trocknungszonen 41 unterschiedliche Temperaturen zur Verfügung gestellt, sodass das Zwischenlaminat 30 in den verschiedenen Trocknungszonen 41 mit verschiedenen Temperaturen, die zwischen 30 °C und 120 °C liegen, beaufschlagt wird.

Das Zwischenlaminat 31 wird derart in die Trocknungsvorrichtung 40 transportiert, dass die wirkstoffhaltige Schicht 31 zu den Luftdüsen 42 weist. Das Substrat 20 ist von den Luftdüsen 42 abgewandt angeordnet.

Nach dem Durchlaufen der Trocknungsvorrichtung 40 liegt ein getrocknetes Zwischenlaminat 32 vor. Dieses getrocknete Zwischenlaminat 32 wird mit Hilfe von Transportrollen 29 zu Laminierwalzen 45 und 45' transportiert. Die Laminierwalze 45 weist eine Rotationsrichtung R₄ auf, wobei die Laminierwalze 45 im Uhrzeigersinn dreht. Die Laminierwalze 45' weist die Rotationsrichtung R₅ auf, diese Rotationsrichtung R₅ ist im dargestellten Beispiel entgegen des Uhrzeigersinns. R₄ und R₅ weisen demnach nicht die gleiche Rotationsrichtung auf.

Nach dem Durchlaufen der Laminierwalzen 45 und 45' liegt somit ein getrocknetes und verfestigtes Zwischenlaminat, also das Wirkstofflaminat 33, vor. Dieses getrocknete Wirkstofflaminat 33 kann aufgerollt werden, sodass ein aufgerolltes Wirkstofflaminat 34 zur Verfügung gestellt werden kann.

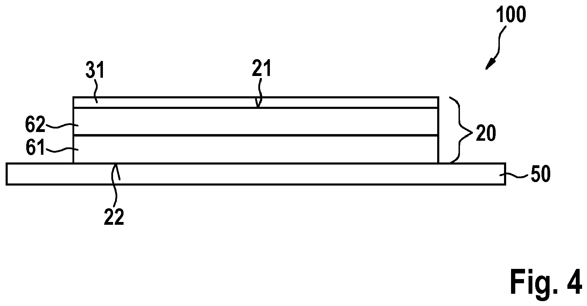

In Fig. 4 wird ein erfindungsgemäßes orales Wirkstofflaminat 100 im Schnitt dargestellt.

Zu erkennen ist eine Trägerfolie 50, die jedoch lediglich in einem Zwischenschritt des erfindungsgemäßen Verfahrens ein Teil des oralen Wirkstofflaminats 100 ist. Das Endprodukt weist keine Trägerfolie 50 auf.

Das Substrat 20 ist aus zwei wirkstofffreien Schichten, nämlich der ersten wirkstofffreien Schicht 61 und der zweiten wirkstofffreien Schicht 62, gebildet. Die Unterseite der ersten wirkstofffreien Schicht 61 bildet die Unterseite 22 des Substrats 20. Die Oberseite der zweiten wirkstofffreien Schicht 62 bildet die Oberseite 21 des Substrats 20. Alternativ könnte das Substrat 20 auch aus wirkstoffhaltigen Schichten gebildet sein. Weiterhin könnte das Substrat 20 aus mindestens einer wirkstoffhaltigen und mindestens einer wirkstofffreien Schicht gebildet sein. Auf der Oberseite 21 des Substrats 20 ist eine wirkstoffhaltige Schicht 31 aufgetragen.

Der Wirkstoff der wirkstoffhaltigen Schicht 31 ist ein Analgetikum, insbesondere Fentanyl und/oder Buprenorphin, das insbesondere als Base oder als Salz vorliegt, und/oder Opioidantagonist, insbesondere Naloxon, sein. Die wirkstofffreien Schichten 61 und 62 können hingegen aus einem Polymer und/oder einem wasserquellbaren Material und/oder einem wasserlöslichen Material gebildet sein.

Die wirkstoffhaltige Schicht 31 weist beispielsweise ein Flächengewicht von 60 g/m² auf. Die wirkstofffreien Substratschichten 61 und 62 weisen hingegen jeweils ein Flächengewicht von 120 g/m² - 140 g/m² auf.

### Bezugszeichenliste

- 10: Produkt
- 11: Trägerfolie
- 12: Matrix
- 13: Oberfläche
- 14: Unterseite
- 15: Tröpfchen
- 20, 20': Substrat
- 21, 21': Oberseite
- 22, 22': Unterseite
- 23, 23': Behälter
- 24, 24': wirkstoffhaltige Masse
- 25, 25': Spalt
- 26, 26': erste drehende Rolle
- 27, 27': zweite drehende Rolle
- 28: dritte drehende Rolle
- 28': Transportrolle
- 29, 29': Transportrolle
- 30, 30': Zwischenlaminat
- 31: wirkstoffhaltige Schicht
- 32: getrocknetes Zwischenlaminat
- 33: Wirkstofflaminat
- 34: aufgerolltes Wirkstofflaminat
- 40: Trocknungsvorrichtung
- 41: Trocknungszone
- 42: Luftdüse
- 45, 45': Laminierwalze
- 50: Trägerfolie
- 61: erste Schicht des Substrats
- 62: zweite Schicht des Substrats
- 100: orales Wirkstofflaminat

- R₁: Rotationsrichtung erste Rolle
- R₂: Rotationsrichtung zweite Rolle
- R₃: Rotationsrichtung dritte Rolle
- R₄: Rotationsrichtung Laminierwalze 45
- R₅: Rotationsrichtung Laminierwalze 45'

## Patentansprüche

1. Verfahren zur Herstellung eines Wirkstofflaminat, insbesondere eines oralen Wirkstofflaminats (100), das mindestens eine wirkstoffhaltige Schicht (31) aufweist, die auf einem Substrat (20) angeordnet ist, wobei das Verfahren folgende Schritte umfasst:
a) Bereitstellen eines Substrats (20) mit einer Oberseite (21) und einer Unterseite (22);
b) Applizieren einer wirkstoffhaltigen Masse (24) in einen Spalt (25), der von einer ersten drehenden Rolle (26) und einer zweiten drehenden Rolle (27) gebildet wird;
c) Transportieren des Substrats (20) mittels einer dritten drehenden Rolle (28) zur zweiten Rolle (27) derart, dass die wirkstoffhaltige Masse (24) von der zweiten Rolle (27) auf die Oberseite (21) des Substrats (20) in Form einer wirkstoffhaltigen Schicht (31) aufgetragen wird;
d) Transportieren eines Zwischenlaminats (30), das aus dem Substrat (20) und der wirkstoffhaltigen Schicht (31) gebildet wird, zu einer Trocknungsvorrichtung (40);
e) Trocknen des Zwischenlaminats (30), insbesondere der wirkstoffhaltigen Schicht (31), wobei
die Rotationsgeschwindigkeit der ersten drehenden Rolle (26) 0,0 m/min - 0,5 m/min beträgt und der zweiten drehenden Rolle (27) 0,8 m/min - 2,3 m/min beträgt und der dritten drehenden Rolle (28) 0,5 m/min - 1,5 m/min beträgt,
und
der Spalt (25) zwischen der ersten drehenden Rolle (26) und der zweiten drehenden Rolle (27) eine Breite von 0,2 mm - 0,9 mm aufweist
und
das Zwischenlaminat (30) in der Trocknungsvorrichtung (40) mit einer Temperatur von 30 °C - 120 °C beaufschlagt wird.

2. Verfahren nach Anspruch 1,
**dadurch gekennzeichnet, dass**
das Substrat (20) auf der Unterseite (22) mit einer Trägerfolie (50), insbesondere lösbar, verbunden ist.

3. Verfahren nach Anspruch 1 oder 2,
**dadurch gekennzeichnet, dass**
beim Trocknen des Zwischenlaminats (30), insbesondere der wirkstoffhaltigen Schicht (31), Lösungsmittel entfernt wird.

4. Verfahren nach einem der Ansprüche 1 bis 3,
**dadurch gekennzeichnet, dass**
das Substrat (20) wirkstofffrei oder wirkstoffhaltig ist.

5. Verfahren nach einem der vorhergehenden Ansprüche,
**dadurch gekennzeichnet, dass**
das Substrat (20) aus einer, insbesondere wirkstofffreien, Schicht, vorzugsweise aus mindestens zwei, insbesondere wirkstofffreien, Schichten (61, 62), gebildet wird.

6. Verfahren nach einem der vorhergehenden Ansprüche,
**dadurch gekennzeichnet, dass**
die erste Rolle (26), die zweite Rolle (27) und die dritte Rolle (28) in der gleichen Rotationsrichtung (R₁; R₂; R₃) drehen.

7. Verfahren nach einem der vorhergehenden Ansprüche,
**gekennzeichnet durch**
f) Führen des getrockneten Zwischenlaminats (32) durch Laminierwalzen (45, 45') und Bilden des Wirkstofflaminats (33),
g) Aufrollen des Wirkstofflaminats (33).

8. Verfahren nach einem der vorhergehenden Ansprüche, insbesondere nach einem der Ansprüche 2 bis 7,
**gekennzeichnet durch**
h) Abrollen des Wirkstofflaminats (34);
i) optionales Entfernen der Trägerfolie (50);
j) Zerteilen des Wirkstofflaminats in eine Vielzahl von Wirkstofflaminatabschnitte.

9. Wirkstofflaminat (100), insbesondere orales Wirkstofflaminat, insbesondere mit einem Verfahren nach einem der Ansprüche 1 bis 8 hergestellt, das mindestens eine wirkstoffhaltige Schicht (31) aufweist, die auf einem Substrat (20) angeordnet ist,
**dadurch gekennzeichnet, dass**
die wirkstoffhaltige Schicht (31) ein von der Dicke und/oder dem Flächengewicht des Substrats (20) unabhängig konstantes Flächengewicht aufweist, wobei
das Substrat (20) wirkstofffrei ist und
der Wirkstoff der wirkstoffhaltigen Schicht (31) ein Analgetikum, insbesondere Fentanyl und/oder Buprenorphin, das insbesondere als Base oder als Salz vorliegt, und/oder Opioidantagonist, insbesondere Naloxon, ist.

10. Wirkstofflaminat (100) nach Anspruch 9,
**dadurch gekennzeichnet, dass**
die wirkstoffhaltige Schicht (31) ein Flächengewicht von 40 g/m² - 100 g/m², insbesondere von 50 g/m² - 70 g/m², insbesondere von 55 g/m² - 65 g/m², insbesondere von 60 g/m², aufweist.

11. Wirkstofflaminat (100) nach einem der Ansprüche 9 bis 10,
**dadurch gekennzeichnet, dass**
das Substrat (20) aus einer, insbesondere wirkstofffreien, Schicht, vorzugsweise aus mindestens zwei, insbesondere wirkstofffreien, Schichten (61, 62), gebildet ist.

12. Wirkstofflaminat (100) nach einem der Ansprüche 9 bis 11, insbesondere nach Anspruch 11,
**dadurch gekennzeichnet, dass**
das Substrat (20) und/oder mindestens eine wirkstofffreie Schicht (61, 62) aus einem Polymer und/oder einem wasserquellbaren Material und/oder einem wasserlöslichen Material gebildet ist.

13. Wirkstofflaminat (100) nach einem der Ansprüche 9 bis 12,
**dadurch gekennzeichnet, dass**
das, insbesondere wirkstofffreie, Substrat (20) ein Flächengewicht von 100 g/m² - 300 g/m², insbesondere von 240 g/m² - 280 g/m², insbesondere von 250 g/m² - 270 g/m², insbesondere von 262 g/m², aufweist.

14. Wirkstofflaminat (100) nach einem der Ansprüche 9 bis 13,
**dadurch gekennzeichnet, dass**
zwei Schichten (61, 62), die das Substrat (20) bilden, jeweils ein Flächengewicht von 80 g/m² - 180 g/m², insbesondere von 100 g/m² - 160 g/m², insbesondere von 120 g/m² - 140 g/m², insbesondere von 131 g/m², aufweisen.

15. Wirkstofflaminat (100) nach einem der Ansprüche 9 bis 14,
**dadurch gekennzeichnet, dass**
auf der zur wirkstoffhaltigen Schicht (31) gegenüberliegenden Seite (22) des Substrats (20) eine Trägerfolie (50) ausgebildet ist.

## Claims

1. A method for producing an active substance laminate, especially an oral active substance laminate (100), having at least one active-substance-containing layer (31), which is arranged on a substrate (20), wherein the method comprises the following steps:
a) providing a substrate (20) having an upper side (21) and an underside (22);
b) applying an active-substance-containing mass (24) in a gap (25) formed by a first rotating roller (26) and a second rotating roller (27);
c) transporting the substrate (20) to the second roller (27) by means of a third rotating roller (28) in such a way that the active-substance-containing mass (24) is applied to the upper side (21) of the substrate (20) by the second roller (27) in the form of an active-substance-containing layer (31);
d) transporting an intermediate laminate (30), formed by the substrate (20) and the active-substance-containing layer (31), to a drying device (40); and
e) drying the intermediate laminate (30), especially the active-substance-containing layer (31), wherein
the rotation speed of the first rotating roller (26) is 0.0 m/min - 0.5 m/min and of the second rotating roller (27) is 0.8 m/min - 2.3 m/min and of the third rotating roller (28) is 0.5 m/min - 1.5 m/min,
and
the gap (25) between the first rotating roller (26) and the second rotating roller (27) has a width of from 0.2 mm - 0.9 mm
and
the intermediate laminate (30) in the drying device (40) is acted on by a temperature of 30 °C - 120 °C.

2. The method according to claim 1,
**characterised in that**
the substrate (20) is connected, especially releasably, on the underside (22) to a carrier film (50).

3. The method according to claim 1 or claim 2,
**characterised in that**
solvent is removed as the intermediate laminate (30), especially the active-substance-containing layer (31), is dried.

4. The method according to any one of claims 1 to 3,
**characterised in that**
the substrate (20) is free from active substance or contains active substance.

5. The method according to any one of the preceding claims,
**characterised in that**
the substrate (20) is formed by one, especially active-substance-free, layer, preferably of at least two, especially active-substance-free, layers (61, 62).

6. The method according to any one of the preceding claims,
**characterised in that**
the first roller (26), the second roller (27) and the third roller (28) rotate in the same rotation direction (R₁; R₂; R₃).

7. The method according to any one of the preceding claims,
**characterised by**
f) guiding the dried intermediate laminate (32) through laminating rollers (45, 45') and forming the active substance laminate (33),
g) rolling up the active substance laminate (33).

8. The method according to any one of the preceding claims, especially according to any one of claims 2 to 7,
**characterised by**
h) unrolling the active substance laminate (34);
i) optionally removing the carrier film (50);
j) dividing the active substance laminate into multiple active substance laminate portions.

9. An active substance laminate (100), especially oral active substance laminate, especially produced by a method according to any one of claims 1 to 8 and having at least one active-substance-containing layer (31), which is arranged on a substrate (20),
**characterised in that**
the active-substance-containing layer (31) has a constant areal density independently of the thickness and/or the areal density of the substrate (20),
wherein
the substrate (20) is free from active substance
and
the active substance of the active-substance-containing layer (31) is an analgesic, especially fentanyl and/or buprenorphine, which especially is present as a base or as a salt, and/or opioid antagonist, especially naloxone.

10. The active substance laminate (100) according to claim 9,
**characterised in that**
the active-substance-containing layer (31) has an areal density of 40 g/m² - 100 g/m², especially of 50 g/m² - 70 g/m², especially of 55 g/m² - 65 g/m², especially of 60 g/m².

11. The active substance laminate (100) according to any one of claims 9 to 10,
**characterised in that**
the substrate (20) is formed by one, especially active-substance-free, layer, preferably of at least two, especially active-substance-free, layers (61, 62).

12. The active substance laminate (100) according to any one of claims 9 to 11, especially according to claim 11,
**characterised in that**
the substrate (20) and/or at least one active-substance-free layer (61, 62) are/is formed from a polymer and/or a material that swells upon contact with water and/or a water-soluble material.

13. The active substance laminate (100) according to any one of claims 9 to 12,
**characterised in that**
the, especially active-substance-free, substrate (20) has an areal density of 100 g/m² - 300 g/m², especially of 240 g/m² - 280 g/m², especially of 250 g/m² - 270 g/m², especially of 262 g/m².

14. The active substance laminate (100) according to any one of claims 9 to 13,
**characterised in that**
two layers (61, 62) which form the substrate (20) each have an areal density of 80 g/m² - 180 g/m², especially of 100 g/m² - 160 g/m², especially of 120 g/m² - 140 g/m², especially of 131 g/m².

15. The active substance laminate (100) according to any one of claims 9 to 14,
**characterised in that**
on the opposite side (22) to the active-substance-containing layer (31) a carrier film (50) is formed on the of the substrate (20).

## Revendications

1. Procédé de fabrication d'un stratifié de principe actif, en particulier d'un stratifié de principe actif oral (100) qui présente au moins une couche contenant un principe actif (31) qui est agencée sur un substrat (20), le procédé comprenant les étapes suivantes consistant à :
a) fournir un substrat (20) avec une face supérieure (21) et une face inférieure (22) ;
b) appliquer une masse contenant un principe actif (24) dans une fente (25) qui est formée par un premier rouleau rotatif (26) et un deuxième rouleau rotatif (27) ;
c) transporter le substrat (20) au moyen d'un troisième rouleau rotatif (28) jusqu'au deuxième rouleau (27) de telle sorte que la masse contenant un principe actif (24) est étalée par le deuxième rouleau (27) sur la face supérieure (21) du substrat (20) sous forme de couche contenant un principe actif (31) ;
d) transporter un stratifié intermédiaire (30) qui est formé à partir du substrat (20) et de la couche contenant un principe actif (31) jusqu'à un dispositif de séchage (40) ;
e) sécher le stratifié intermédiaire (30), en particulier la couche contenant un principe actif (31), dans lequel
la vitesse de rotation du premier rouleau rotatif (26) est de 0,0 m/min à 0,5 m/min, celle du deuxième rouleau rotatif (27) est de 0,8 m/min à 2,3 m/min et celle du troisième rouleau rotatif (28) est de 0,5 m/min à 1,5 m/min
et
la fente (25) entre le premier rouleau rotatif (26) et le deuxième rouleau rotatif (27) présente une largeur de 0,2 mm à 0,9 mm
et
le stratifié intermédiaire (30) dans le dispositif de séchage (40) est soumis à une température de 30 °C à 120 °C.

2. Procédé selon la revendication 1,
**caractérisé en ce que**
le substrat (20) est relié à un film support (50), en particulier détachable, sur la face inférieure (22).

3. Procédé selon la revendication 1 ou 2,
**caractérisé en ce que**
lors du séchage du stratifié intermédiaire (30), en particulier de la couche contenant un principe actif (31), un solvant est éliminé.

4. Procédé selon l'une quelconque des revendications 1 à 3,
**caractérisé en ce que**
le substrat (20) est dépourvu de principe actif ou contient un principe actif.

5. Procédé selon l'une quelconque des revendications précédentes,
**caractérisé en ce que**
le substrat (20) est formé à partir d'une couche, en particulier dépourvue de principe actif, de préférence à partir de deux couches, en particulier dépourvues de principe actif, (61, 62).

6. Procédé selon l'une quelconque des revendications précédentes,
**caractérisé en ce que**
le premier rouleau (26), le deuxième rouleau (27) et le troisième rouleau (28) tournent dans la même direction de rotation (R₁ ; R₂ ; R₃).

7. Procédé selon l'une quelconque des revendications précédentes, **caractérisé par** les étapes consistant à
f) faire passer le stratifié intermédiaire séché (32) à travers des rouleaux de stratification (45, 45') et former le stratifié de principe actif (33),
g) enrouler le stratifié de principe actif (33).

8. Procédé selon l'une quelconque des revendications précédentes, en particulier selon l'une quelconque des revendications 2 à 7, **caractérisé par** les étapes consistant à
h) dérouler le stratifié de principe actif (34) ;
i) en option, retirer le film support (50) ;
j) découper le stratifié de principe actif en une multiplicité de sections de stratifié de principe actif.

9. Stratifié de principe actif (100), en particulier stratifié de principe actif oral, en particulier fabriqué avec un procédé selon l'une quelconque des revendications 1 à 8, qui présente au moins une couche contenant un principe actif (31) qui est agencée sur un substrat (20),
**caractérisé en ce que** :
la couche contenant un principe actif (31) présente un poids surfacique constant, indépendamment de l'épaisseur et/ou du poids surfacique du substrat (20), dans lequel
le substrat (20) est dépourvu de principe actif, et
le principe actif de la couche contenant un principe actif (31) est un antalgique, en particulier du fentanyl et/ou de la buprénorphine, se présentant en particulier comme base ou comme sel, et/ou un antagoniste opioïde, en particulier du naloxone.

10. Stratifié de principe actif (100) selon la revendication 9,
**caractérisé en ce que**
la couche contenant un principe actif (31) présente un poids surfacique de 40 g/m² à 100 g/m², en particulier de 50 g/m² à 70 g/m², en particulier de 55 g/m² à 65 g/m², en particulier de 60 g/m².

11. Stratifié de principe actif (100) selon l'une quelconque des revendications 9 à 10,
**caractérisé en ce que**
le substrat (20) est formé à partir d'une couche, en particulier dépourvue de principe actif, de préférence à partir de deux couches, en particulier dépourvues de principe actif, (61, 62).

12. Stratifié de principe actif (100) selon l'une quelconque des revendications 9 à 11, en particulier selon la revendication 11,
**caractérisé en ce que**
le substrat (20) et/ou au moins une couche dépourvue de principe actif (61, 62) sont formés d'un polymère et/ou d'un matériau gonflant dans l'eau et/ou d'un matériau soluble dans l'eau.

13. Stratifié de principe actif (100) selon l'une quelconque des revendications 9 à 12,
**caractérisé en ce que**
le substrat (20), en particulier dépourvu de principe actif, présente un poids surfacique de 100 g/m² à 300 g/m², en particulier de 240 g/m² à 280 g/m², en particulier de 250 g/m² à 270 g/m², en particulier de 262 g/m².

14. Stratifié de principe actif (100) selon l'une quelconque des revendications 9 à 13,
**caractérisé en ce que**
deux couches (61, 62) qui forment le substrat (20) présentent chacune un poids surfacique de 80 g/m² à 180 g/m², en particulier de 100 g/m² à 160 g/m², en particulier de 120 g/m² à 140 g/m², en particulier de 131 g/m².

15. Stratifié de principe actif (100) selon l'une quelconque des revendications 9 à 14,
**caractérisé en ce que**
un film support (50) est réalisé sur la face (22) du substrat (20) opposée à la couche contenant un principe actif (31).
